(19) Europäisches Patentamt / European Patent Office / Office européen des brevets

(11) **EP 2 893 937 B1**

(12) **EUROPEAN PATENT SPECIFICATION**

(45) Date of publication and mention
of the grant of the patent:
**01.07.2020 Bulletin 2020/27**

(51) Int Cl.:
*A61K 39/102* (2006.01)   *A61P 31/04* (2006.01)
*C12N 15/09* (2006.01)

(21) Application number: **13834953.5**

(22) Date of filing: **06.09.2013**

(86) International application number:
**PCT/JP2013/074075**

(87) International publication number:
**WO 2014/038662 (13.03.2014 Gazette 2014/11)**

(54) **DNA VACCINE AGAINST PSEUDOTUBERCULOSIS IN MARINE FISH**

DNA-IMPFSTOFFE GEGEN PSEUDOTUBERCULOSIS BEI MEERESFISCHEN

VACCIN A ADN CONTRE PSEUDOTUBERCULOSIS DANS UN POISSON MARIN

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **10.09.2012 JP 2012198719**

(43) Date of publication of application:
**15.07.2015 Bulletin 2015/29**

(73) Proprietor: **National University Corporation
Tokyo University of Marine Science
And Technology
Minato-ku
Tokyo 108-8477 (JP)**

(72) Inventors:
• **HIRONO, Ikuo**
 **Tokyo 108-8477 (JP)**
• **KONDO, Hidehiro**
 **Tokyo 108-8477 (JP)**
• **YAMASHITA, Kozue**
 **Tokyo 108-8477 (JP)**

(74) Representative: **Cohausz & Florack
Patent- & Rechtsanwälte
Partnerschaftsgesellschaft mbB
Bleichstraße 14
40211 Düsseldorf (DE)**

(56) References cited:
EP-A2- 1 538 210    WO-A2-01/10459
WO-A2-2005/014629    JP-A- H09 285 291

• Ikuo Hirono ET AL: "Identification of major antigenic proteins of Pasteurella piscicida", Microbial Pathogenesis, 1 January 1997 (1997-01-01), pages 371-380, XP055242724, ENGLAND DOI: 10.1006/mpat.1997.0165 Retrieved from the Internet: URL:http://ac.els-cdn.com/S0882401097901650/1-s2.0-S0882401097901650-main.pdf?_tid=24c44cd8-beb0-11e5-b9e2-00000aab0f02&acdnat=1453210124_f29cbd62665cf76d78b0e1d0c943fc 7e [retrieved on 2016-01-19]
• ANA DO VALE ET AL: "AIP56, a novel plasmid-encoded virulence factor of Photobacterium damselae subsp. piscicida with apoptogenic activity against sea bass macrophages and neutrophils", MOLECULAR MICROBIOLOGY, vol. 58, no. 4, 1 November 2005 (2005-11-01), pages 1025-1038, XP055106980, ISSN: 0950-382X, DOI: 10.1111/j.1365-2958.2005.04893.x
• A.D. Le Breton: "Vaccines in Mediterranean aquaculture: practice and needs", The use of veterinary drugs and vaccines in Mediterranean aquaculture, 1 January 2009 (2009-01-01), pages 147-154, XP055253773, Retrieved from the Internet: URL:http://om.ciheam.org/om/pdf/a86/00801068.pdf [retrieved on 2016-02-29]
• G Kurath: "Biotechnology and DNA vaccines for aquatic animals", Revue scientifique et technique (International Office of Epizootics), 1 April 2008 (2008-04-01), pages 175-196, XP055253775, France Retrieved from the Internet: URL:http://web.oie.int/boutique/extrait/15 kurath175196.pdf [retrieved on 2016-02-29]

**(Cont. next page)**

- NAKA H ET AL.: 'Random sequencing of genomic DNA of Photobacterium damselae subsp. piscicida' FISHERIES SCIENCE vol. 71, November 2005, pages 1209 - 1216, XP055242719
- HIRONO I ET AL.: 'Identification of major antigenic proteins of Pasteurella piscicida.' MICROB PATHOG. vol. 23, no. 6, December 1997, pages 371 - 380, XP055242724
- DAVIES M N ET AL.: 'Harnessing bioinformatics to discover new vaccines.' DRUG DISCOV TODAY. vol. 12, no. 9-10, 06 April 2007, pages 389 - 395, XP022063933
- RAPPUOLI R ET AL.: 'Reverse vaccinology and genomics.' SCIENCE vol. 302, no. 5645, 24 October 2003, page 602, XP055242733
- DATABASE CAPLUS [Online] 26 January 2010 'THE GENOME OF PHOTOBACTERIUM DAMSELAE PISCICIDA AND THE DEVELOPMENT OF VACCINES AGAINST FISH PASTEURELLOSIS', XP055256749 Retrieved from STN Database accession no. 2009:1386706 & IT 1 370 040 B 26 January 2010

- GILCHUK PAVLO ET AL: "Discovering protective CD8 T cell epitopes-no single immunologic property predicts it!", CURRENT OPINION IN IMMUNOLOGY, ELSEVIER, OXFORD, GB, vol. 34, 6 February 2015 (2015-02-06), pages 43-51, XP029605146, ISSN: 0952-7915, DOI: 10.1016/J.COI.2015.01.013

**Description**

TECHNICAL FIELD

The present invention relates to a DNA vaccine for inducing protective immunity against an infection to fish of *Photobacterium damselae subsp. piscicida,* which is a causative bacterium of pseudotuberculosis.

The term "pseudotuberculosis" as used herein means pseudotuberculosis caused by the infection of *P. damselae subsp. piscicida.* Therefore, not only pseudotuberculosis developed in Amberjacks, but also pasteurellosis developed caused by the same causative bacterium in fish other than yellowtails [for example, fish belonging to Perciformes (Japanese black porgy, red seabream, Redspotted grouper, and the like), fish belonging to Osmeriformes (ayu sweetfish, and the like), and fish belonging to Tetraodontiformes (black scraper, and the like)] is included.

BACKGROUND ART

In the aquaculture industry of many aquatic organisms such as fish and shellfish, viral disease and bacterial diseases in an aquaculture area, which is a closed system, have become serious problems, because individuals are present at a high density, and thus, the impact of these infections is large.

Pseudotuberculosis (also known as pasteurellosis) was first reported as a cause disease of a mass mortality of white perch (Roccus americanus) in the Chesapeake Bay, USA in 1963 (Non-patent literature 1). In Japan, its occurrence was observed in aquacultured yellowtail (0-year-old fish) in the southwest of Shikoku in 1968, and was spread in aquacultured yellowtail in most of western Japan in the next year, in 1969. Furthermore, pseudotuberculosis occurred in various fish, such as Japanese black porgy, red seabream, Redspotted grouper, ayu sweetfish, and black scraper, as well as yellowtails, and since this disease has a very strong contagiousness, it has become a problem that threatens marine aquaculture (Non-patent literature 1).

*P. damselae subsp. piscicida* belongs to the genus Photobacterium, and is a gram-negative, facultative anaerobic, non-motile, short-rod-shaped bacterium (0.6 to 1.2 $\mu$m$\times$0.8 to 2.6 $\mu$m). Its growth optimum temperature is 25 to 30°C, its optimum pH is 7.5 to 8.0, and its optimum sodium chloride concentration is 2 to 3%. It is sensitive to ampicillin, oxolinic acid, florfenicol, and the like. The symptoms of this disease are characterized by a formation of small white spots having a diameter of about 1 mm in the kidney and spleen. Colonies of the bacterium are placed in the small white spots, and many of the spots form nodules surrounded by fibrous tissue. The formation of these bacterial colonies is based on a formation of bacterial balls in capillaries or interstitial tissue, by resisting the intracellular digestion by phagocytes, and causing the growth within phagocytes (Non-patent literature 2) .

An inactivated vaccine against the disease has been already approved in Japan, but the activated vaccine is difficult to induce cellular immunity. However, the development of vaccines having a high inducibility of cellular immunity is not progressing (Patent literatures 1 and 2)

Vaccines are generally used for the prevention or treatment of bacterial infections. Vaccines include inactivated vaccines (Japanese encephalitis, Weil's disease, and the like), toxoids (tetanus, diphtheria, and the like), attenuated vaccines (BCG, polio, and the like), recombinant vaccines (hepatitis B virus, and the like), and the like. The inactivated vaccines, and the toxoids prepared by detoxifying an exotoxin induce antibodies against these vaccines, and are relatively safe vaccines. The recombinant vaccines do not contain impurities, and therefore, they are believed to be safer vaccines, in comparison with the inactivated vaccines.

However, although these vaccines can induce antibody production, it is a disadvantage that cellular immunity is difficult to be induced. Further, with respect to the inactivated vaccines and the attenuated vaccines, it is industrially necessary to obtain a large amount of protein as the antigen, and it is essential to grow an appropriate pathogen. Furthermore, the immune effects acquired by the attenuated vaccines are often maintained for a long period of time, but side effects and risks are pointed out. With respect to the inactivated vaccines and the recombinant vaccines, it is believed that the persistence of the antigen is short in a host, and it needs an adjuvant and the like. With respect to these conventional vaccines, since refrigerated storage is necessary during a period from manufacturing to inoculation into subjects, there is a problem that an increase in cost and a decrease in potency occur.

With the recent progress in the research and development of vaccines, a new type of vaccine (DNA vaccine), which brings immune induction by administering a plasmid DNA encoding an immunogenic protein, has been developed, and has been improving the disadvantages of the conventional vaccines, as described below. That is to say, advantages of the DNA vaccine are: that since it can strongly induce not only humoral immune response, but also cellular immunity, it can impart a protective activity against infectious diseases; that it can be highly purified; that since it is stable at room temperature or even at high temperatures, refrigerated storage is not essential, and it can be stored for a long period of time; that it can be easily modified by genetic engineering techniques; that the time spent on vaccine development can be shortened; and the like.

It is known that an immune response in rainbow trout and flounder can be stimulated by intramuscular injection of a gene encoding glycoprotein, as a constituent protein, of Rhabdoviruses (Non-patent literature 3). Further, there is a report of DNA vaccines for rainbow trout and flounder (Non-patent literature 4). However, there is no report of DNA vaccines in other fish.

Patent literature 3 describes a derivative of a 55 kDA extracellular protein from *Photobacterium damselae* subsp. *Piscicida* is the basis for a vaccine against *Photobacterium* infection, and thereby protects fish from pasteurellosis.

Patent literature 4 describes a method of immunization of aquaculture species by introducing DNA expression systems into the aquaculture species.

Non-patent literature 5 describes two different antigenic protein-coding clones (PPA1 and PPA2) that were isolated using anti-Pasteurella *piscicida* rabbit serum from a genomic DNA library of *P. piscicida* strain KP9038.

CITATION LIST

PATENT LITERATURE

[Patent literature 1] JP H09-176043 A
[Patent literature 2] JP 2002-003400 A
[Patent literature 3] WO 2005/014 62 9 A2
[Patent literature 4] EP 1 538 210 A2

NON-PATENT LITERATURE

[Non-patent literature 1] S. F. Snieszko et al., Bacteriology, (USA), 1964, vol. 88, p. 1814-1814
[Non-patent literature 2] Hisashi Wakabayashi and Kiyokuni Muroga ed., Gyokai-rui no Kansen-sho·Kiseichu-sho (Infectious and parasitic diseases of fish and shellfish), (KOUSEISHA KOUSEIKAKU Co., Ltd.), 2004, p. 206-211
[Non-patent literature 3] P. Boudinot et al., Virology, (USA), 1998, vol. 249, p. 297-306
[Non-patent literature 4] McLauchlan et al., Fish and shellfish Immunology, (UK), 2003, vol. 15, p. 39-50
[Non-patent literarure 5] Ikuo Hirone et al. "Identification of major antigenic proteins of Pasteurella piscicida", Microbial Pathogenesis, 1 January 1997, p. 371 - 380

SUMMARY OF INVENTION

TECHNICAL PROBLEM

An object of the present invention is to provide a DNA vaccine for fish for inducing protective immunity against pseudotuberculosis. The invention is defined by the claims. Any other aspects or embodiments set forth herein not falling within the scope of the claims are for information only.

SOLUTION TO PROBLEM

The inventors of the present invention conducted intensive studies on vaccines effective against pseudotuberculosis in marine fish, and as a result, plasmid DNAs containing a gene encoding a lipoprotein (ppa1: SEQ ID NO: 1), a DegQ serine protease (ppa2: SEQ ID NO: 3), or an outer membrane protein A precursor (ppars1: SEQ ID NO: 5) of *P. damselae subsp. piscicida* were administered, as a mixture or separately, to Japanese flounder and Greater amberjack, and found that these DNAs had immune effects against pseudotuberculosis in marine fish, and that the expression levels of immune-related genes were increased. Furthermore, these sequences were modified in accordance with a codon usage in flounder to prepare artificial genes (SEQ ID NOs: 7, 9, and 11), and plasmid DNAs containing these sequences were administered in a similar fashion. As a result, it was confirmed that each exhibited high immunological protection effects, and the present invention was completed.

The present invention relates to:

[1] A DNA vaccine for fish, characterized by

- imparting immunity against pseudotuberculosis caused by *Photobacterium damselae subsp. piscicida ;*
- comprising, as an active ingredient,

a DNA comprising a nucleotide sequence encoding an immunogenic polypeptide against *Photobacterium damselae* subsp. *piscicida;* a DNA comprising a nucleotide sequence prepared by modifying the sequence based on a codon usage in Japanese flounder;
or an expression vector comprising the DNA,

wherein the immunogenic polypeptide is

(1) a polypeptide consisting of the amino acid sequence of SEQ ID NO: 2;
(2) a polypeptide comprising the amino acid sequence of SEQ ID NO: 2; or
(3) a homologous polypeptide with immunogenicity against. *Photobacterium damselae* subsp. *piscicida,* comprising an amino acid sequence having a 90% identity or more with the amino acid sequence of SEQ ID NO: 2; or

an immunogenic partial fragment of any one of the polypeptides (1) to (3), whereby the length of the partial fragment is not limited, so long as a polypeptide encoded by the partial nucleotide sequence can induce immunity, including humoral immunity and cellular immunity, against *Photobacterium damselae* subsp. *piscicida* in the living body,

[2] the DNA vaccine for fish according to [1], wherein the nucleotide sequence is

(1) the nucleotide sequence of SEQ ID NO: 1 or 7; or
(2) a nucleotide sequence having an 80% homology or more with the nucleotide sequence of SEQ ID NO: 1 or 7, and encoding a polypeptide with immunogenicity against *Photobacterium damselae* subsp. *piscicida;* or

an immunogenic partial fragment of any one of the nucleotide sequences (1) and (2), whereby the length of the partial fragment is not limited, so long as a polypeptide encoded by the nucleotide sequence can induce immunity, including humoral immunity and cellular immunity, against *Photobacterium damselae* subsp. *piscicida* in the living body,

[3] the DNA vaccine for fish according to [1], wherein the expression vector is a plasmid comprising the nucleotide sequence of SEQ ID NO: 1 or 7,

[4] a DNA vaccine for fish according to any one of [1] to [3] for use in a method of preventing pseudotuberculosis, wherein the method is characterized by administering the DNA vaccine for fish according to any one of [1] to [3] to fish,

[5] the DNA vaccine for fish for use according to [4], wherein the fish is fish belonging to Perciformes, Tetraodontiformes, or Osmeriformes.

Further, the present invention relates to:
a DNA comprising a nucleotide sequence encoding an immunogenic polypeptide against *Photobacterium damselae subsp. piscicida,* or an expression vector comprising the DNA, for a DNA vaccine for fish, or for the prevention of pseudotuberculosis, and use of a DNA comprising a nucleotide sequence encoding an immunogenic polypeptide against *Photobacterium damselae subsp. piscicida,* or an expression vector comprising the DNA, in the manufacture of a DNA vaccine for fish.

ADVANTAGEOUS EFFECTS OF INVENTION

According to the DNA vaccine for fish of the present invention, the immunity against pseudotuberculosis caused by *P. damselae* subsp. *piscicida* can be imparted. More particularly, the DNA vaccine for fish of the present invention can induce an immune response (including a humoral immune response and a cellular immune response) against infectious diseases of *P. damselae subsp. piscicida,* or pseudotuberculosis caused by the infection of *P. damselae subsp. piscicida,* and therefore, it is effective in the prevention of the infection of *P. damselae subsp. piscicida,* or the prevention of pseudotuberculosis. For example, plasmids comprising the nucleotide sequence of SEQ ID NO: 1 or 7, which may be used as an active ingredient of the DNA vaccine for fish of the present invention, are effective for the active ingredient of the DNA vaccine, on the basis of a protection test against the infection of *P. damselae subsp. piscicida* in marine fish, and the results in which remarkable increases in the expression levels of immune-related genes were confirmed, and therefore, the plasmids can be expected in the prevention of the infection of P. *damselae* subsp. *piscicida* in marine fish.

BRIEF DESCRIPTION OF THE DRAWINGS

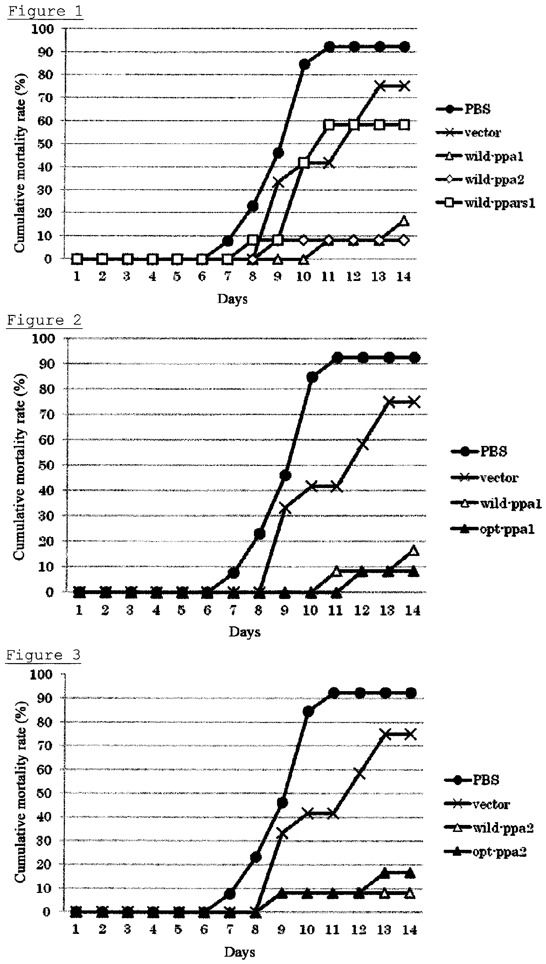

[Fig. 1] Fig. 1 is a graph showing, with a cumulative mortality after the challenge of *P. damselae subsp. piscicida* strain TUMSAT-PPE05-02 (immersion at $1.0 \times 10^5$ cfu/mL), the change in a cumulative mortality rate in Japanese flounder treated with DNA vaccines (wild-ppal, wild-ppa2, or wild-ppars1), in an immersion group at $1.0 \times 10^5$ cfu/mL.
[Fig. 2] Fig. 2 is a graph showing, with a cumulative mortality after the challenge of *P. damselae subsp. piscicida* strain TUMSAT-PPE05-02 (immersion at $1.0 \times 10^5$ cfu/mL), the change in a cumulative mortality rate in Japanese flounder treated with DNA vaccines (wild-ppal or opt-ppa1), in an immersion group at $1.0 \times 10^5$ cfu/mL.
[Fig. 3] Fig. 3 is a graph showing, with a cumulative mortality after the challenge of *P. damselae subsp. piscicida* strain TUMSAT-PPE05-02 (immersion at $1.0 \times 10^5$ cfu/mL), the change in a cumulative mortality rate in Japanese flounder treated with DNA vaccines (wild-ppa2 or opt-ppa2), in an immersion group at $1.0 \times 10^5$ cfu/mL.

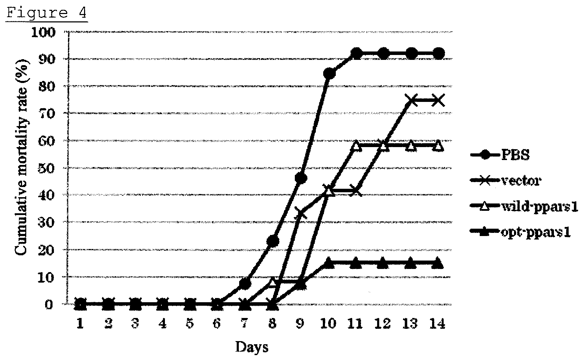

[Fig. 4] Fig. 4 is a graph showing, with a cumulative mortality after the challenge of *P. damselae subsp. piscicida* strain TUMSAT-PPE05-02 (immersion at $1.0 \times 10^5$ cfu/mL), the change in a cumulative mortality rate in Japanese flounder treated with DNA vaccines (wild-ppa3 or opt-ppa3), in an immersion group at $1.0 \times 10^5$ cfu/mL.

DESCRIPTION OF EMBODIMENTS

The DNA vaccine for fish of the present invention is not limited, so long as it is a DNA construct comprising at least one (i.e., one or more) nucleotide sequence encoding an immunogenic polypeptide against *P. damselae subsp. piscicida*. Examples of the DNA vaccine for fish of the present invention include

(a) a DNA comprising a nucleotide sequence encoding an immunogenic polypeptide against *P. damselae subsp. piscicida,* and
(b) an expression vector comprising the DNA (a).

The DNA (a) can further contain various regulatory sequences required for the expression of the immunogenic polypeptide, and the expression vector (b) can also contain such regulatory sequences.
The term "immunogenic polypeptide against pseudotuberculosis" as used herein means a polypeptide capable of inducing immunity (including humoral immunity and cellular immunity) against *P. damselae subsp. piscicida* in the living body.
The immunogenic polypeptide against *P. damselae subsp. piscicida* is not limited, so long as it is a polypeptide capable of inducing immunity (including humoral immunity and cellular immunity) against *P. damselae subsp. piscicida* in the living body. The immunogenic polypeptide is a polypeptide encoded by ppa1 of *P. damselae subsp. piscicida,* or its partial fragment, and a polypeptide consisting of the amino acid sequence of SEQ ID NO: 2 or its partial fragment.
Examples of the immunogenic polypeptide further include:

(1) a polypeptide comprising the amino acid sequence of SEQ ID NO: 2;
(2) a modified polypeptide with immunogenicity against *Photobacterium damselae subsp. piscicida,* comprising an amino acid sequence in which one or plural amino acids are deleted, substituted, or added in the amino acid sequence of SEQ ID NO: 2; and
(3) a homologous polypeptide with immunogenicity against *Photobacterium damselae subsp. piscicida,* comprising an amino acid sequence having an 90% or more identity with the amino acid sequence of SEQ ID NO: 2; and a partial fragment of any one of the polypeptides (1) to (3).

The DNA vaccine for fish of the present invention may be a DNA vaccine capable of inducing only one immunogenic polypeptide (ppa1 or only the modified or homologous polypeptide thereof), or a DNA vaccine capable of inducing two or more immunogenic polypeptides (preferably a combination of ppa1 or the modified or homologous polypeptide thereof with one or more polypeptides selected from the polypeptides encoded by ppa1, ppa2, and ppars1, and the modified or homologous polypeptides thereof). Examples of the latter include a combination of the ppa1 protein (or its modified or homologous polypeptide) and the ppa2 protein (or its modified or homologous polypeptide), a combination of the ppa1 protein (or its modified or homologous polypeptide) and the ppars1 protein (or its modified or homologous polypeptide), and a combination of the ppa1 protein (or its modified or homologous polypeptide), the ppa2 protein (or its modified or homologous polypeptide), and the ppars1 protein (or its modified or homologous polypeptide).
The "modified polypeptide" as used herein means a protein in which one or more (for example, 1 to several, preferably 1 to 10, more preferably 1 to 5, still more preferably 1 to 3, still more preferably 1 to 2, and most preferably 1) amino acids are modified (for example, deleted, substituted, and/or added) in the amino acid sequence of a certain SEQ ID number, and immunity can still be imparted to the fish to which the present invention can be applied.

The term "identity in amino acid sequences" as used herein means an identity calculated by comparing and analyzing two amino acid sequences using a computer analysis software (SDC software), and regarding the two amino acid sequences as the same when a same kind of amino acid is located at the same position.

As the nucleotide sequence encoding the immunogenic polypeptide, which may be used in the present invention, the above-mentioned nucleotide sequences encoding each immunogenic polypeptide may be exemplified. Examples of the nucleotide sequence include the polypeptides comprising the amino acid sequence of SEQ ID NO: 2, the modified polypeptides or the homologous polypeptides, and partial fragments thereof.

As the nucleotide sequence,

(1) the nucleotide sequence of SEQ ID NO: 1, or
(2) a nucleotide sequence having an 80% homology or more with the nucleotide sequence of SEQ ID NO: 1, or, and encoding a polypeptide with immunogenicity against *Photobacterium damselae subsp. piscicida;*
or a partial fragment of any one of the nucleotide sequences (1) and (2), is preferable.

The term "homology in nucleotide sequences" as used herein means a value calculated by comparing and analyzing two nucleotide sequences using a computer analysis software (SDC software), and regarding the two nucleotide sequences as the same when the same kind of nucleotide is located at the same position.

The length of the partial fragment is not limited, so long as a polypeptide encoded by the partial nucleotide sequence can induce immunity (including humoral immunity and cellular immunity) against P. *damselae subsp. piscicida* in the living body.

The nucleotide sequence encoding the immunogenic polypeptide may be a naturally-occurring sequence, a completely synthetic sequence, or a partially synthetic sequence utilizing a naturally-occurring sequence.

The nucleotide sequence encoding the immunogenic polypeptide (for example, a lipoprotein), which may be used in the present invention, can be obtained from, for example, *P. damselae subsp. piscicida.* As a typical method of obtaining the nucleotide sequence used in the present invention, a conventional method in the field of genetic engineering, for example, a screening method using an appropriate DNA probe designed based on the information of a partial amino acid sequence, may be exemplified.

The expression vector used in the present invention is not limited, so long as it is a vector capable of expressing in cells of fish. The expression vector used in the present invention may be constructed on the basis of a self-replicating vector, such as plasmid, which exists as an extra-chromosomal independent body and does not depend on the chromosomal replication. The expression vector may be one which may be incorporated into the genome of a host microorganism, when it is transformed with the expression vector, and which may be replicated together with the chromosome into which it is incorporated. The expression vector, which may be used in the present invention, may be constructed in accordance with procedures and methods widely used in the field of genetic engineering.

As the transcription regulatory sequence, which may be used in the present invention, for example, a constitutive promoter, an inducible or regulatory promoter, a tissue-specific promoter, a promoter derived from the gene of an expressed antigen, or the like may be exemplified, but the transcription regulatory sequence is not limited to these promoters, so long as it can be expressed in cells of fish. Examples of the constitutive promoter include a promoter sequence derived from cytomegalovirus (CMV), or strong promoters such as Rous sarcoma virus (RSV), simian virus-40 (SV-40), muscle β-actin promoter, herpes simplex virus (HSV), or the like. As the tissue-specific promoter, for example, a thymidine kinase promoter may be exemplified. Examples of the inducible or regulatory promoter include a growth hormone regulatory promoter, a promoter which is under the control of a lac operon sequence, or a zinc-inducible metallothionein promoter. The transcription regulatory sequence may be operably linked to the nucleotide sequence encoding the immunogenic polypeptide (i.e., so as to regulate the expression of the nucleotide sequence).

The regulatory sequence can contain an expression regulatory sequence including the promoter DNA sequence (for example, the inducible or constitutive promoter) and, if desired, a copy or more copies selected from an enhancer element, an intron sequence for splicing a transcription or polyadenylation signal (for example, simian virus-40 (SV-40) or bovine growth hormone-derived), or an immunostimulatory DNA sequence known as a CpG motif.

The expression vector may contain, if desired, for example, a bacterial replication origin sequence, or a selection marker for screening, such as an antibiotic resistance (for example, kanamycin) gene or a non-antibiotic resistance gene (for example, a β-galactosidase gene).

It is known that an oligonucleotide containing unmethylated CpG nucleotides activates the immune system (A. Krieg et al., Nature, 1995, 374, 546-549). A certain CpG motif is immunostimulatory against a B cell or T cell response, depending on a flanking sequence, and preferentially stimulates a certain species. The copy of the CpG motif in the DNA expression vector functions as an adjuvant which induces an immune response against the expressed protein. The CpG motif, i.e., a DNA elongation portion containing the CpG dinucleotide in the specified sequence, can be selected from lengths of about 5 to 40 base pairs. Plural motifs can be inserted into noncoding regions of the expression vector.

In the case where humoral response is desired, a CpG motif which stimulates the secretion of cytokines which are known to stimulate a CD8+ T cell response is preferable.

The fish to which the present invention is applied is not limited, so long as it may be infected with *P. damselae subsp. piscicida.* Examples of the fish include fish belonging to Perciformes (yellowtail, greater amberjack, Japanese black porgy, red seabream, Redspotted grouper, and the like), fish belonging to Osmeriformes (ayu sweetfish, and the like), and fish belonging to Tetraodontiformes (black scraper, and the like).

Examples of a method of inoculating the DNA vaccine include an oral administration, an intramuscular injection, an intraperitoneal injection, an administration using a gene gun, and an immersion, and the intramuscular injection or the administration using a gene gun is preferable. The administration using a gene gun is a method in which gold particles having a diameter of about 1 μm is coated with the plasmid, and the particles are shot in skin, cells, or a tissue of a subject with a dedicated instrument, using highpressure helium gas, in the manner of an air gun. The administration using a gene gun has superior features, in comparison with the intramuscular injection, that equivalent immune effects can be achieved with DNA at an amount of 1/100 to 1/1000, and that it has superior reproducibility to the intramuscular injection.

An adjuvant enhances an immune reaction against an antigen by the stimulation of the immune system, and is generally added to the vaccine as an auxiliary. For example, an aluminum compound, a polynucleotide, a bacterial component of bacteria, and the like are known to typical adjuvants, but many of them are not sufficient to obtain the effects of the present invention. In particular, since the action of adjuvants is widely effective in antigen substances, there are risks that the antigen stimulation of impurities contained in antigens is increased or that harmful side effects are raised, and there is a problem that it is necessary to sufficiently consider to the purity of the antigen used. Under the circumstances, it has been reported that, for example, IL-1β is effective as an adjuvant (J. Y. Scheerlinck, Genetic adjuvants for DNA Vaccine, 19, 2647-2656, 2001). In the present invention, a plasmid into which an IL-1β gene is introduced so that it can be expressed in the living body of fish (for example, flounder) can be prepared, and fish can be inoculated with the plasmid together with the vaccine of the present invention.

The immune mechanism demonstrates a variety of physiological functions, while cells which play various roles mutually regulate the functions. The activation of the immune system can be analyzed using, as an index, the expression levels of a T cell antigen receptor (TCR), a major histocompatibility complex (MHC), or an immunoglobulin (Ig), which is present on the cell surface of T cells and B cells, which are factors that play an important role in the biological defense. In the present invention, for example, after flounder is inoculated with the DNA vaccine against pseudotuberculosis, in order to analyze the activation of the biological defense mechanism in the living body of fish, for example, a change in the expression levels of TCR, MHC, and Ig can be quantitatively confirmed by carrying out a real-time PCR, and the activation of the immune system can be analyzed.

The real-time PCR is a detection technique by monitoring the gene amplification process by PCR in real-time, using a fluorescence detection device, and profiling the PCR reaction curve. When a sample to be tested is amplified by PCR, the accurate amount of DNA can be calculated by examining the number of PCR cycles that reach the exponential increase curve. The real-time PCR can be carried out using, for example, TaqMan (Perkin-Elmer) and iCycler (BioRad).

In the real-time PCR, two kinds of primers, a primer for real-time PCR (SG) and a primer for standard DNA (SG200), are used. The primer for real-time PCR (SG) is designed so that the amplicon is short (60 to 100 bp), and the GC content is low. In the case where the primer is designed at the 3' UTR portion, a gene-specific primer can be designed relatively easily and accurately. Furthermore, there is a method of designing the primer on a computer using a software (for example, primer express : PE Biosystems Japan Ltd.). The primer for standard DNA (SG200) is designed on the outside of the primer designed in real-time PCR. In the case where genes are compared with each other, it is preferable to adjust the amplicon.

In the preparation of standard DNA, specific primers (SG200) corresponding to each gene to be measured are used to carry out PCR (total volume: 50 μL) until it reaches a plateau. In the PCR, after a reaction at 95°C for 2 minutes is carried out, a cycle consisting of reactions at 95°C for 30 seconds, at 55°C for 30 seconds, and at 72°C for 1 minute are repeated 30 times. Subsequently, the resulting PCR products are subjected to a column (for example, Microcon-PCR Centrifugal Filter Devices; MILLIPORE) to remove excess primers, and the concentration is measured. The copy number is calculated from the Avogadro constant (1 mol = $6.022 \times 10^{23}$ molecules). Next, each PCR product is diluted to prepare a dilution series (21 steps, copy number: $10^{13}$ to $10^{-7}$), and PCR was carried out using the primers (SG) which are designed on the inside. This PCR is carried out at a cycle number of 14 cycles so that the amplification occurs exponentially. The resulting PCR produces are subjected to agarose electrophoresis. Since the exponential amplification occurs at the next 5 steps after bands are completely invisible, these 5 steps are used, as standard DNA, in the true real-time PCR.

EXAMPLES

The present invention now will be further illustrated by, but is by no means limited to, the following Examples.

<<Example 1: Isolation of ppa1 gene, ppa2 gene, and ppars1 gene derived from *P. damselae subsp. piscicida*>>

(1) Preparation of PCR primers

The following PCR primers were prepared in accordance with a conventional method, based on the DNA nucleotide sequences corresponding to a ppa1 gene (SEQ ID NO: 1), a ppa2 gene (SEQ ID NO: 3), and a pparsl gene (SEQ ID NO: 5) derived from *P. damselae subsp. piscicida.*
ppa1-forward = 5' CGGAATTCACCATGAATCGTAAAGTAACTA 3'(SEQ ID NO: 13)
ppa1-reverse = 5' CCGCTCGAGCTTAGTGTAAGAACCAC 3'(SEQ ID NO: 14)
ppa2-forward = 5' CGGAATTCACCATGAGAAAACCTCTGCTTG 3'(SEQ ID NO: 15)
ppa2-reverse = 5' CCGCTCGAGACGCATGATTAAATACA 3'(SEQ ID NO: 16)
ppars1-forward = 5' CGGAATTCACCATGTCTAAAGTTCGTTATG 3'(SEQ ID NO: 17)
ppars1-reverse = 5' CCGCTCGAGTTCAGCAAGAACTTGAG 3'(SEQ ID NO: 18)

(2) Preparation of DNA vaccines

*P. damselae subsp. piscicida* strain TUMSAT-PPE05-02 was inoculated in an HI liquid medium supplemented with 2% NaCl, and cultivated with shaking at 25°C overnight. Cells were collected, and suspended in a solvent for extraction (0.5% SDS and 0.01.6 mg/mL protease K), and treated at 37°C for 1 hour. To the liquid, 100 $\mu$L of 5 mol/L NaCl and 84 $\mu$L of CTAB/NaCl were added and well mixed, and treated at 65°C for 10 minutes. The resulting mixture was purified using an equal volume of isopropanol/chloroform, 600 $\mu$L of PCI, and 99% ethanol in turn. The supernatant was discarded, and genomic DNA as a pellet was dried, and dissolved in 30 $\mu$L of a TE buffer.
The extracted genomic DNA was used as a template to perform a PCR, using commercially available PCR reaction reagents (ExTaq DNA polymerase; Takara Shuzo Co., Ltd.), in accordance with a manual attached thereto, and the PCR primers prepared in Example 1(1), in accordance with a conventional method. In the PCR, 5 $\mu$L of attached 10xbuffer, 4 $\mu$L of dNTP, 1 $\mu$L of forward and reverse primers (25 pmol/$\mu$L), 0.5 $\mu$L of DNA polymerase (5 units/$\mu$L), 1 $\mu$L of DNA (10 ng), and 37.5 $\mu$L of sterile water were mixed to adjust to 50 $\mu$L, and after heating at 95°C for 5 minutes, a cycle composed of reactions at 95°C for 30 seconds, at 53°C for 30 seconds, and at 72°C for 1 minute was repeated 30 times, and a reaction at 72°C for 5 minutes was performed. The resulting products were subjected to agarose gel electrophoresis, and it was confirmed visually that DNA fragments of about 0.2, 1.4, and 1.0 kbp were amplified.

(3) Analysis of DNA nucleotide sequences

The amplified DNA fragments were inserted into a pGEM-T Eazy vector (Promega), and samples were prepared by a dideoxy method, using a fluorescent labeled M13 primer (Nisshinbo Industries Inc.) and a commercially available sequencing kit (Thermo Sequence Fluorescent Labeled Primer Cycle Sequencing Kit with 7-deaza-dGTP; amersham pharmacia bitech), and the nucleotide sequences were determined using a DNA sequencer (DNA sequencer model 4000; LI-COR). According to this analysis, the DNA fragments amplified by PCR contained open reading frames consisting of 249 bp (ppa1), 1356 bp (ppa2), and 996 bp (ppars1), respectively (SEQ ID NOs: 1, 3, and 5). The proteins predicted from these ORFs had 89, 462, and 341 amino acid residues.

<<Example 2: Preparation of modified ppa1 gene, ppa2 gene, and ppars1 gene based on codon usage in Japanese flounder

(1) Preparation of artificial genes using modified sequences

There are plural codons which specify an amino acid in any species, and the codon usages thereof are different from one another. A ppa1 gene (SEQ ID NO: 7), a ppa2 gene (SEQ ID NO: 9), and a ppars1 gene (SEQ ID NO: 11) in which the nucleotide sequences were modified based on the codon usages in *P. damselae subsp. piscicida* and Japanese flounder were artificially synthesized, and were inserted into a plasmid.

<<Example 3: Preparation of plasmids wild-ppal, wild-ppa2, wild-pparsl, opt-ppa1, opt-ppa2, and opt-pparsl»

The plasmids which had been used in the analysis of DNA nucleotide sequences in Examples 1(3) and 2(1) were treated with EcoRI and XhoI. The ppa1 gene, the ppa2 gene, and the ppars1 gene derived from *P. damselae subsp. piscicida* strain TUMSAT-PPE05-02, and the artificial ppa1 gene, the artificial ppa2 gene, and the artificial ppars1 gene, which were excised from each vector, were inserted between the EcoRI and XhoI recognition sites of a multiple cloning site at the downstream of a sequence encoding a human cytomegalovirus promoter region of a pcDNA3.1/myc-his vector (Invitrogen), to prepare plasmids wild-ppal, wild-ppa2, wild-pparsl, opt-ppa1, opt-ppa2, and opt-ppars1.

<<Example 4: Introduction of plasmid DNAs to Japanese flounder>>

The wild-ppal, wild-ppa2, wild-pparsl, opt-ppa1, opt-ppa2, and opt-ppars1 (10.0 μg/flounder) were administered into the muscle, together with 100 μL of phosphate-buffered saline (PBS), using a syringe with a 29G needle.

<<Example 5: Protection test against infection in Japanese flounder>>

Young Japanese flounder (total length: about 8 cm, average fish weight: 10.0 g) were used in each test group. Test fish were bred at an average water temperature of 19.0°C in a water bath of 60 L while circulating artificial seawater.
In accordance with the method of Example 4, 10.0 μg of wild-ppal, wild-ppa2, wild-pparsl, opt-ppa1, opt-ppa2, and opt-ppars1 were separately administered to the flounder. As controls, 100 μL of PBS and 10.0 μg of pcDNA3.1 vector were separately administered. After 30 days from the administration, a culture medium obtained by cultivating *P. damselae subsp. piscicida* strain TUMSAT-PPE05-02 in the HI liquid medium supplemented with 2% NaCl, in a similar fashion to that of Example 1(2), was diluted to $1.0 \times 10^5$ cfu/mL using artificial seawater, and an immersion infection was carried out for 30 minutes. The test groups in which a protection test against infection was carried out were as follows:

Test group 1: wild-ppal 10.0 μg/flounder + $1.0 \times 10^5$ cfu/mL *P. damselae subsp. piscicida* strain TUMSAT-PPE05-02
Comparative Test group 2: wild-ppa2 10.0 μg/flounder + $1.0 \times 10^5$ cfu/mL *P. damselae subsp. piscicida* strain TUMSAT-PPE05-02
Comparative Test group 3: wild-ppars1 10.0 μg/flounder + $1.0 \times 10^5$ cfu/mL *P. damselae subsp. piscicida* strain TUMSAT-PPE05-02
Test group 4: opt-ppa1 10.0 μg/flounder + $1.0 \times 10^5$ cfu/mL *P. damselae subsp. piscicida* strain TUMSAT-PPE05-02
Comparative Test group 5: opt-ppa2 10.0 μg/flounder + $1.0 \times 10^5$ cfu/mL P. *damselae subsp. piscicida* strain TUMSAT-PPE05-02
Comparative Test group 6: opt-ppars1 10.0 pg/flounder + $1.0 \times 10^5$ cfu/mL P. *damselae subsp. piscicida* strain TUMSAT-PPE05-02
Test group 7: pcDNA3.1/myc-his vector 10.0 μg/flounder + $1.0 \times 10^5$ cfu/mL P. *damselae subsp. piscicida* strain TUMSAT-PPE05-02
Test group 8: PBS 100 μL/flounder + $1.0 \times 10^5$ cfu/mL P. *damselae subsp. piscicida* strain TUMSAT-PPE05-02

After the infection of *P. damselae subsp. piscicida* strain TUMSAT-PPE05-02, the flounder were observed for 14 days, and "a cumulative mortality rate of flounder (death population/test population) × 100 (%)" was calculated in test groups 1 to 8. The results are shown in Figures 1, 2, 3, and 4, and Table 1. The "RPS" in the Table is an abbreviation of "relative percent survival", and is calculated by the following equation:

$$RPS = \{1 - (X/C)\} \times 100$$

[The symbol "X" is "a mortality rate in each vaccination group (%)", and "C" is "a mortality rate in the negative control group"]
The effects of vaccines were judged by comparison.
As a result, the mortality rates in Test groups 1 to 6 were respectively about 17%, 8%, 58%, 8%, 17%, and 15%, whereas those in Test groups 7 and 8 were 75% and 90%, and therefore, it was revealed that the wild-ppal, wild-ppa2, wild-pparsl, opt-ppa1, opt-ppa2, and opt-ppars1 were effective in the protection against the infection of *P. damselae subsp. piscicida* strain TUMSAT-PPE05-02, that is to say, the vaccine effects were revealed.

Table 1

| Test group | | Cumulative mortality rate (%) | RPS against Test group 8 |
|---|---|---|---|
| 1 | wild-ppal | 16.7 | 81.9 |
| 2 * | wild-ppa2 | 8.33 | 91 |
| 3 * | wild-ppars1 | 58.3 | 36.8 |
| 4 | opt-ppa1 | 8.33 | 91 |
| 5 * | opt-ppa2 | 16.7 | 81.9 |

(continued)

| Test group | | Cumulative mortality rate (%) | RPS against Test group 8 |
|---|---|---|---|
| 6 * | opt-ppars1 | 15.4 | 83.3 |
| 7 | Vector | 75 | 18.7 |
| 8 | PBS | 92.3 | - |
| * comparative | | | |

INDUSTRIAL APPLICABILITY

The DNA vaccine of the present invention can be applied for the prevention of pseudotuberculosis in marine fish.

Although the present invention has been described with reference to specific embodiments, various changes and modifications obvious to those skilled in the art are possible without departing from the scope of the appended claims.

FREE TEXT IN SEQUENCE LISTING

Features of "Artificial Sequence" are described in the numeric identifier <223> in the Sequence Listing. More particularly, the nucleotide sequences of SEQ ID NOs: 7, 9, and 11 are modified sequences derived from *P. damselae subsp. piscicida.* The nucleotide sequence of SEQ ID NO: 13 is primer ppa1-forward. The nucleotide sequence of SEQ ID NO: 14 is primer ppa1-reverse. The nucleotide sequence of SEQ ID NO: 15 is primer ppa2-forward. The nucleotide sequence of SEQ ID NO: 16 is primer ppa2-reverse. The nucleotide sequence of SEQ ID NO: 17 is primer ppars1-forward. The nucleotide sequence of SEQ ID NO: 18 is primer ppars1-reverse.

**Claims**

1. A DNA vaccine for fish, **characterized by**

   - imparting immunity against pseudotuberculosis caused by *Photobacterium damselae subsp. piscicida;*
   - comprising, as an active ingredient,

     a DNA comprising a nucleotide sequence encoding an immunogenic polypeptide against *Photobacterium damselae subsp. piscicida;*
     a DNA comprising a nucleotide sequence prepared by modifying the sequence based on a codon usage in Japanese flounder;
     or an expression vector comprising the DNA,

   wherein the immunogenic polypeptide is

     (1) a polypeptide consisting of the amino acid sequence of SEQ ID NO: 2;
     (2) a polypeptide comprising the amino acid sequence of SEQ ID NO: 2; or
     (3) a homologous polypeptide with immunogenicity against *Photobacterium damselae subsp. piscicida,* comprising an amino acid sequence having a 90% identity or more with the amino acid sequence of SEQ ID NO: 2; or
     an immunogenic partial fragment of any one of the polypeptides (1) to (3), whereby the length of the partial fragment is not limited, so long as a polypeptide encoded by the partial nucleotide sequence can induce immunity, including humoral immunity and cellular immunity, against *Photobacterium damselae subsp. piscicida* in the living body.

2. The DNA vaccine for fish according to claim 1, wherein the nucleotide sequence is

     (1) the nucleotide sequence of SEQ ID NO: 1 or 7; or
     (2) a nucleotide sequence having an 80% homology or more with the nucleotide sequence of SEQ ID NO: 1 or 7, and encoding a polypeptide with immunogenicity against *Photobacterium damselae subsp. piscicida;* or
     an immunogenic partial fragment of any one of the nucleotide sequences (1) and (2), whereby the length of the partial fragment is not limited, so long as a polypeptide encoded by the partial nucleotide sequence can induce immunity, including humoral immunity and cellular immunity, against *Photobacterium damselae subsp. piscicida* in the living body.

3. The DNA vaccine for fish according to claim 1, wherein the expression vector is a plasmid comprising the nucleotide sequence of SEQ ID NO: 1 or 7.

4. A DNA vaccine for fish according to any one of claims 1 to 3 for use in a method of preventing pseudotuberculosis, wherein the method is **characterized by** administering the DNA vaccine for fish according to any one of claims 1 to 3 to fish.

5. The DNA vaccine for fish for use according to claim 4, wherein the fish is fish belonging to Perciformes, Tetraodontiformes, or Osmeriformes.


**Patentansprüche**

1. Ein DNA-Impfstoff für Fische, **dadurch gekennzeichnet, dass** er:

     - Immunität gegen von *Photobacterium damselae subsp. piscicida* verursachte Pseudotuberkulose verleiht und
     - als Wirkstoff:

     eine DNA, welche eine Nucleotidsequenz, die ein immunogenes Polypeptid gegen *Photobacterium damselae subsp. piscicida* codiert, umfasst,
     eine DNA, welche eine Nucleotidsequenz umfasst, die durch Modifizieren der Sequenz, das auf einer Codonverwendung in der Japanischen Flunder beruht, hergestellt worden ist,
     oder einen die DNA enthaltenden Expressionsvektor umfasst,

wobei das immunogene Polypeptid:

(1) ein Polypeptid, das aus der Aminosäuresequenz mit der SEQ ID NO: 2 besteht,
(2) ein Polypeptid, das die Aminosäuresequenz mit der SEQ ID NO: 2 umfasst, oder
(3) ein homologes Polypeptid mit Immunogenität gegen *Photobacterium damselae subsp. piscicida,* das eine Aminosäuresequenz mit einer Identität von 90 % oder höher mit der Aminosäuresequenz mit der SEQ ID NO: 2 umfasst, oder
ein immunogenes Teilfragment eines der Polypeptide (1) bis (3) ist, wobei die Länge des Teilfragments nicht beschränkt ist, solange ein von der Teilnucleotidsequenz codiertes Polypeptid Immunität, einschließlich humoraler Immunität und zellulärer Immunität, gegen *Photobacterium damselae subsp. piscicida* in dem lebenden Körper auslösen kann.

2. Der DNA-Impfstoff für Fische nach Anspruch 1, wobei die Nucleotidsequenz:

(1) die Nucleotidsequenz mit der SEQ ID NO: 1 oder 7 ist; oder
(2) eine Nucleotidsequenz, die eine Homologie von 80 % oder höher mit der Nucleotidsequenz mit der SEQ ID NO: 1 oder 7 besitzt und ein Polypeptid mit Immunogenität gegen *Photobacterium damselae subsp. piscicida* codiert, ist; oder
ein immunogenes Teilfragment einer der Nucleotidsequenzen (1) und (2) ist, wobei die Länge des Teilfragments nicht beschränkt ist, solange ein von der Teilnucleotidsequenz codiertes Polypeptid Immunität, einschließlich humoraler Immunität und zellulärer Immunität, gegen *Photobacterium damselae subsp. piscicida* in dem lebenden Körper auslösen kann.

3. Der DNA-Impfstoff für Fische nach Anspruch 1, wobei der Expressionsvektor ein Plasmid ist, das die Nucleotidsequenz mit der SEQ ID NO: 1 oder 7 umfasst.

4. Ein DNA-Impfstoff für Fische nach einem der Ansprüche 1 bis 3 zur Verwendung in einem Verfahren für die Verhütung der Pseudotuberkulose, wobei das Verfahren **dadurch gekennzeichnet ist, dass** der DNA-Impfstoff für Fische nach einem der Ansprüche 1 bis 3 Fischen verabreicht wird.

5. Der DNA-Impfstoff für Fische zur Verwendung nach Anspruch 4, wobei der Fisch ein Fisch ist, der zu Perciformes, Tetraodontiformes oder Osmeriformes gehört.

**Revendications**

1. Vaccin à ADN pour poisson, **caractérisé en ce qu'**il

- confère l'immunité contre la pseudotuberculose causée par *Photobacterium damselae subsp. piscicida ;*
- comprend, comme ingrédient actif,

un ADN comprenant une séquence nucléotidique codant un polypeptide immunogène vis-à-vis de *Photobacterium damselae subsp. piscicida ;*
un ADN comprenant une séquence nucléotidique préparée en modifiant la séquence sur la base d'un usage des codons chez la limande japonaise ;
ou un vecteur d'expression comprenant l'ADN,

le polypeptide immunogène étant

(1) un polypeptide constitué de la séquence d'acides aminés de SEQ ID n° : 2 ;
(2) un polypeptide comprenant la séquence d'acides aminés de SEQ ID n° : 2 ; ou
(3) un polypeptide homologue ayant une immunogénicité vis-à-vis de *Photobacterium damselae subsp. piscicida,* comprenant une séquence d'acides aminés ayant 90 % d'identité ou plus vis-à-vis de la séquence d'acides aminés de SEQ ID n° : 2 ; ou
un fragment immunogène partiel de l'un quelconque des polypeptides (1) à (3), moyennant quoi la longueur du fragment partiel n'est pas limitée, tant qu'un polypeptide codé par la séquence nucléotidique partielle peut induire l'immunité, comprenant l'immunité humorale et l'immunité cellulaire, contre *Photobacterium damselae subsp. piscicida* dans le corps vivant.

**2.** Vaccin à ADN pour poisson selon la revendication 1, la séquence nucléotidique étant

(1) la séquence nucléotidique de SEQ ID n° : 1 ou 7 ; ou
(2) une séquence nucléotidique ayant une homologie de 80 % ou plus vis-à-vis de la séquence nucléotidique de SEQ ID n° : 1 ou 7, et codant un polypeptide ayant une immunogénicité vis-à-vis de *Photobacterium damselae subsp. piscicida,* ou
un fragment immunogène partiel de l'une quelconque des séquences nucléotidiques (1) et (2), moyennant quoi la longueur du fragment partiel n'est pas limitée, tant qu'un polypeptide codé par la séquence nucléotidique partielle peut induire l'immunité, comprenant l'immunité humorale et l'immunité cellulaire, contre *Photobacterium damselae subsp. piscicida* dans le corps vivant.

**3.** Vaccin à ADN pour poisson selon la revendication 1, le vecteur d'expression étant un plasmide comprenant la séquence nucléotidique de SEQ ID n° : 1 ou 7.

**4.** Vaccin à ADN pour poisson selon l'une quelconque des revendications 1 à 3 pour l'utilisation dans un procédé de prévention de la pseudotuberculose, le procédé étant **caractérisé par** l'administration du vaccin à ADN pour poisson selon l'une quelconque des revendications 1 à 3 au poisson.

**5.** Vaccin à ADN pour poisson pour l'utilisation selon la revendication 4, le poisson étant un poisson appartenant aux perciformes, tétraodontiformes, ou osmériformes.

Figure 1

Figure 2

Figure 3

Figure 4

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- JP H09176043 A
- JP 2002003400 A
- WO 2005014629 A2
- EP 1538210 A2
- JP 2012198719 A

### Non-patent literature cited in the description

- **S. F. SNIESZKO et al.** *Bacteriology,* 1964, vol. 88, 1814-1814
- Gyokai-rui no Kansen-sho·Kiseichu-sho (Infectious and parasitic diseases of fish and shellfish). KOUSEISHA KOUSEIKAKU Co., Ltd, 2004, 206-211
- **P. BOUDINOT et al.** *Virology,* 1998, vol. 249, 297-306
- **MCLAUCHLAN et al.** *Fish and shellfish Immunology,* 2003, vol. 15, 39-50
- **IKUO HIRONE et al.** Identification of major antigenic proteins of Pasteurella piscicida. *Microbial Pathogenesis,* 01 January 1997, 371-380
- **A. KRIEG et al.** *Nature,* 1995, vol. 374, 546-549
- **J. Y. SCHEERLINCK.** *Genetic adjuvants for DNA Vaccine,* 2001, vol. 19, 2647-2656

SEQUENCE LISTING

<110> National University Corporation Tokyo University of Marine Science and Technology

<120> DNA vaccine for pseudotuberculosis of marine fish

<130> KYD-918

<150> JP 2012-198719
<151> 2012-09-10

<160> 18

<170> PatentIn version 3.5

<210> 1
<211> 303
<212> DNA
<213> Photobacterium damselae subsp. piscicida

<220>
<221> CDS
<222> (19)..(288)

<400> 1

```
agtgtggtgg aattcacc atg aat cgt aaa gta act atc cta gct ggt gtt          51
                     Met Asn Arg Lys Val Thr Ile Leu Ala Gly Val
                     1               5                   10

att cta agt gca act cta atg ggt tgt tca agc tca agc gaa atg cag          99
Ile Leu Ser Ala Thr Leu Met Gly Cys Ser Ser Ser Glu Met Gln
            15                  20                  25

caa cta act aac aaa gtt gac gca ctt tct gat caa gta agc gct cta         147
Gln Leu Thr Asn Lys Val Asp Ala Leu Ser Asp Gln Val Ser Ala Leu
            30                  35                  40

caa gcc aac aga tca act agt ggc gct gtt aac gac gct cgt gca gca         195
Gln Ala Asn Arg Ser Thr Ser Gly Ala Val Asn Asp Ala Arg Ala Ala
            45                  50                  55

tct gac gca gct tac caa gaa gca atg cgt gct aac cag cgc atc gac         243
Ser Asp Ala Ala Tyr Gln Glu Ala Met Arg Ala Asn Gln Arg Ile Asp
60                  65                  70                  75

aac atc cgt ggt tct tac act aag cat cat cac cat cac cac tag           288
Asn Ile Arg Gly Ser Tyr Thr Lys His His His His His His
                     80                  85

ctcgagtcta gaggg                                                        303
```

<210> 2
<211> 89
<212> PRT
<213> Photobacterium damselae subsp. piscicida

<400> 2

```
Met Asn Arg Lys Val Thr Ile Leu Ala Gly Val Ile Leu Ser Ala Thr
1               5                   10                  15

Leu Met Gly Cys Ser Ser Ser Ser Glu Met Gln Gln Leu Thr Asn Lys
            20                  25                  30

Val Asp Ala Leu Ser Asp Gln Val Ser Ala Leu Gln Ala Asn Arg Ser
            35                  40                  45

Thr Ser Gly Ala Val Asn Asp Ala Arg Ala Ala Ser Asp Ala Ala Tyr
    50                  55                  60

Gln Glu Ala Met Arg Ala Asn Gln Arg Ile Asp Asn Ile Arg Gly Ser
65                  70                  75                  80

Tyr Thr Lys His His His His His His
            85
```

<210> 3
<211> 1419
<212> DNA
<213> Photobacterium damselae subsp. piscicida

<220>
<221> CDS
<222> (19)..(1404)

<400> 3

```
agtgtggtgg aattcacc atg aga aaa cct ctg ctt gtt tta agt gca ctg      51
                    Met Arg Lys Pro Leu Leu Val Leu Ser Ala Leu
                     1           5                      10

gct tta ggt att agt tca gtt gtc acc ccg atg acg gca act gcg gcg      99
Ala Leu Gly Ile Ser Ser Val Val Thr Pro Met Thr Ala Thr Ala Ala
             15                  20                  25

tta cct acc gcc gtt aat aat caa caa gta cct agc ctt gcg cca atg     147
Leu Pro Thr Ala Val Asn Asn Gln Gln Val Pro Ser Leu Ala Pro Met
             30                  35                  40

ctt gag caa gta aca cca gct gta gta agc att gca gtt gaa ggt aaa     195
Leu Glu Gln Val Thr Pro Ala Val Val Ser Ile Ala Val Glu Gly Lys
         45                  50                  55

cac gtt tcc aaa cag cgc cta cct gag gct tat cgc ttt ttc ttt ggt     243
His Val Ser Lys Gln Arg Leu Pro Glu Ala Tyr Arg Phe Phe Phe Gly
 60                  65                  70                  75

cca gat ttt cct acc gag caa gta cga gaa caa cca ttt agg ggg tta     291
Pro Asp Phe Pro Thr Glu Gln Val Arg Glu Gln Pro Phe Arg Gly Leu
                 80                  85                  90

ggc tcg ggg gtt atc att gac gct aaa aaa ggg tat gtc gtt acc aat     339
Gly Ser Gly Val Ile Ile Asp Ala Lys Lys Gly Tyr Val Val Thr Asn
             95                 100                 105

aac cat gtt att gat ggc gca gat acc atc aaa gta cag ctt tct gac     387
Asn His Val Ile Asp Gly Ala Asp Thr Ile Lys Val Gln Leu Ser Asp
```

                    110                          115                          120

```
gga cgt gag ata aaa gca aag ctg ctt ggt acc gat aaa atg tct gat          435
Gly Arg Glu Ile Lys Ala Lys Leu Leu Gly Thr Asp Lys Met Ser Asp
    125                 130                 135

att gcg ttg ctg caa tta gaa gat gcc aaa gat ctt acg gct atc aaa          483
Ile Ala Leu Leu Gln Leu Glu Asp Ala Lys Asp Leu Thr Ala Ile Lys
140                 145                 150                 155

ctc gct gat tct gat aat ctt cgc gta ggc gat ttc gcc gtt gct atc          531
Leu Ala Asp Ser Asp Asn Leu Arg Val Gly Asp Phe Ala Val Ala Ile
                160                 165                 170

ggt aac cca ttt ggc cta gga caa aca gta aca tca ggt att gtt tcc          579
Gly Asn Pro Phe Gly Leu Gly Gln Thr Val Thr Ser Gly Ile Val Ser
                175                 180                 185

gct ctt ggt cgt agt ggt tta aat atc gag aac ttc gaa aac ttt atc          627
Ala Leu Gly Arg Ser Gly Leu Asn Ile Glu Asn Phe Glu Asn Phe Ile
                190                 195                 200

caa act gat gcg cca atc aat agc ggt aac tca ggt ggt gcc tta gta          675
Gln Thr Asp Ala Pro Ile Asn Ser Gly Asn Ser Gly Gly Ala Leu Val
    205                 210                 215

aac tta aac ggt gag ttg att ggt att aat acc gcg att ctt gcg cct          723
Asn Leu Asn Gly Glu Leu Ile Gly Ile Asn Thr Ala Ile Leu Ala Pro
220                 225                 230                 235

aac ggt ggt aac gtc ggt atc ggt ttt gca atc cct gct aat atg gtg          771
Asn Gly Gly Asn Val Gly Ile Gly Phe Ala Ile Pro Ala Asn Met Val
                240                 245                 250

aaa aac tta aca gat caa ctt atc gaa ttt ggt caa gtt aag cgc ggt          819
Lys Asn Leu Thr Asp Gln Leu Ile Glu Phe Gly Gln Val Lys Arg Gly
                255                 260                 265

gtt ctg ggt gtt act ggt ggt gag cta acc tct gag cta gct gaa acc          867
Val Leu Gly Val Thr Gly Gly Glu Leu Thr Ser Glu Leu Ala Glu Thr
    270                 275                 280

ttt ggt tat aaa aca aat cac ggc gcg ttt gta aac caa gta ctt cct          915
Phe Gly Tyr Lys Thr Asn His Gly Ala Phe Val Asn Gln Val Leu Pro
    285                 290                 295

gaa ggc tct gcg gca aaa gca ggt cta aaa gca ggt gat att att gtt          963
Glu Gly Ser Ala Ala Lys Ala Gly Leu Lys Ala Gly Asp Ile Ile Val
300                 305                 310                 315

tca gtc aat aat aag ccg att cgt act ttc agt gaa tta cgc gca aaa         1011
Ser Val Asn Asn Lys Pro Ile Arg Thr Phe Ser Glu Leu Arg Ala Lys
                320                 325                 330

att gta act cta ggt gct ggt aag aaa gta aca cta ggc cta att cgc         1059
Ile Val Thr Leu Gly Ala Gly Lys Lys Val Thr Leu Gly Leu Ile Arg
                335                 340                 345

gat ggt aaa gag ctg aat gtt cct gtg act tta gaa gca gca aaa cag         1107
Asp Gly Lys Glu Leu Asn Val Pro Val Thr Leu Glu Ala Ala Lys Gln
                350                 355                 360

act caa gtt aaa gct gat gat ctg cat gaa agc tta gcc ggt gct gaa         1155
```

14

```
Thr Gln Val Lys Ala Asp Asp Leu His Glu Ser Leu Ala Gly Ala Glu
    365             370             375

ttt gct aat act agc cca gaa gat aaa gtt cac gga gtt aaa gta acc    1203
Phe Ala Asn Thr Ser Pro Glu Asp Lys Val His Gly Val Lys Val Thr
380             385             390             395

gag ctg aac aag caa tct att gcg gct cgc tat ggc ttg aag aag ggc    1251
Glu Leu Asn Lys Gln Ser Ile Ala Ala Arg Tyr Gly Leu Lys Lys Gly
            400             405             410

gat atc att att ggt cta aac cgt cag cca att aaa aac ctt ggc gaa    1299
Asp Ile Ile Ile Gly Leu Asn Arg Gln Pro Ile Lys Asn Leu Gly Glu
            415             420             425

ctt cgt aag gcg ctt gag aaa aaa cca aac gta ctt gca atg gaa gtt    1347
Leu Arg Lys Ala Leu Glu Lys Lys Pro Asn Val Leu Ala Met Glu Val
            430             435             440

aaa cgt ggt gat agc gta ctg tat tta atc atg cgt cat cat cac cat    1395
Lys Arg Gly Asp Ser Val Leu Tyr Leu Ile Met Arg His His His His
    445             450             455

cac cac tag ctcgagtcta gaggg                                       1419
His His
460
```

<210> 4
<211> 461
<212> PRT
<213> Photobacterium damselae subsp. piscicida

<400> 4

```
Met Arg Lys Pro Leu Leu Val Leu Ser Ala Leu Ala Leu Gly Ile Ser
1               5               10              15

Ser Val Val Thr Pro Met Thr Ala Thr Ala Ala Leu Pro Thr Ala Val
            20              25              30

Asn Asn Gln Gln Val Pro Ser Leu Ala Pro Met Leu Glu Gln Val Thr
            35              40              45

Pro Ala Val Val Ser Ile Ala Val Glu Gly Lys His Val Ser Lys Gln
    50              55              60

Arg Leu Pro Glu Ala Tyr Arg Phe Phe Phe Gly Pro Asp Phe Pro Thr
65              70              75              80

Glu Gln Val Arg Glu Gln Pro Phe Arg Gly Leu Gly Ser Gly Val Ile
            85              90              95

Ile Asp Ala Lys Lys Gly Tyr Val Val Thr Asn Asn His Val Ile Asp
            100             105             110
```

```
Gly Ala Asp Thr Ile Lys Val Gln Leu Ser Asp Gly Arg Glu Ile Lys
    115                 120             125

Ala Lys Leu Leu Gly Thr Asp Lys Met Ser Asp Ile Ala Leu Leu Gln
    130                 135             140

Leu Glu Asp Ala Lys Asp Leu Thr Ala Ile Lys Leu Ala Asp Ser Asp
145                 150             155                 160

Asn Leu Arg Val Gly Asp Phe Ala Val Ala Ile Gly Asn Pro Phe Gly
            165                 170             175

Leu Gly Gln Thr Val Thr Ser Gly Ile Val Ser Ala Leu Gly Arg Ser
            180                 185             190

Gly Leu Asn Ile Glu Asn Phe Glu Asn Phe Ile Gln Thr Asp Ala Pro
        195                 200             205

Ile Asn Ser Gly Asn Ser Gly Gly Ala Leu Val Asn Leu Asn Gly Glu
    210                 215             220

Leu Ile Gly Ile Asn Thr Ala Ile Leu Ala Pro Asn Gly Gly Asn Val
225                 230             235                 240

Gly Ile Gly Phe Ala Ile Pro Ala Asn Met Val Lys Asn Leu Thr Asp
            245                 250             255

Gln Leu Ile Glu Phe Gly Gln Val Lys Arg Gly Val Leu Gly Val Thr
            260                 265             270

Gly Gly Glu Leu Thr Ser Glu Leu Ala Glu Thr Phe Gly Tyr Lys Thr
        275                 280             285

Asn His Gly Ala Phe Val Asn Gln Val Leu Pro Glu Gly Ser Ala Ala
    290                 295             300

Lys Ala Gly Leu Lys Ala Gly Asp Ile Ile Val Ser Val Asn Asn Lys
305                 310             315                 320

Pro Ile Arg Thr Phe Ser Glu Leu Arg Ala Lys Ile Val Thr Leu Gly
            325                 330             335

Ala Gly Lys Lys Val Thr Leu Gly Leu Ile Arg Asp Gly Lys Glu Leu
        340                 345             350

Asn Val Pro Val Thr Leu Glu Ala Ala Lys Gln Thr Gln Val Lys Ala
    355                 360             365
```

```
Asp Asp Leu His Glu Ser Leu Ala Gly Ala Glu Phe Ala Asn Thr Ser
    370             375             380

Pro Glu Asp Lys Val His Gly Val Lys Val Thr Glu Leu Asn Lys Gln
385             390             395             400

Ser Ile Ala Ala Arg Tyr Gly Leu Lys Lys Gly Asp Ile Ile Ile Gly
                405             410             415

Leu Asn Arg Gln Pro Ile Lys Asn Leu Gly Glu Leu Arg Lys Ala Leu
            420             425             430

Glu Lys Lys Pro Asn Val Leu Ala Met Glu Val Lys Arg Gly Asp Ser
        435             440             445

Val Leu Tyr Leu Ile Met Arg His His His His His His
    450             455             460
```

<210> 5
<211> 1059
<212> DNA
<213> Photobacterium damselae subsp. piscicida

<220>
<221> CDS
<222> (19)..(1044)

<400> 5

```
agtgtggtgg aattcacc atg tct aaa gtt cgt tat gtt ctt ccg ttg gca        51
                    Met Ser Lys Val Arg Tyr Val Leu Pro Leu Ala
                     1           5                   10

ctt cta att tca ggt gtc gct aac gca gca gca gat aac cca tgg tat        99
Leu Leu Ile Ser Gly Val Ala Asn Ala Ala Ala Asp Asn Pro Trp Tyr
            15                  20                  25

gcg ggt ttc cga gtt ggt gct act cac tat aat gat att tct gta aat       147
Ala Gly Phe Arg Val Gly Ala Thr His Tyr Asn Asp Ile Ser Val Asn
        30                  35                  40

ggt gtg gac agt aat tct aca ttt gac cgc gat gat atg ggc ggt ggt       195
Gly Val Asp Ser Asn Ser Thr Phe Asp Arg Asp Asp Met Gly Gly Gly
        45              50                  55

ttg ttt gca ggt tac aac gta act cct tgg ttc gcg gtt gaa act ggc       243
Leu Phe Ala Gly Tyr Asn Val Thr Pro Trp Phe Ala Val Glu Thr Gly
60                  65                  70                  75

tac act tgg cta ggc cgt gct gag ttt gat aat aac tac aaa atg cga       291
Tyr Thr Trp Leu Gly Arg Ala Glu Phe Asp Asn Asn Tyr Lys Met Arg
                80                  85                  90

gtt gat cag caa gcg atc gat ctt gtt ggt aaa ttt aca tgg cat gca       339
Val Asp Gln Gln Ala Ile Asp Leu Val Gly Lys Phe Thr Trp His Ala
                95                  100                 105
```

```
aca gac tat atg ggt ctt tat gcg aaa ctg ggt ggt gca tac tac ttc          387
Thr Asp Tyr Met Gly Leu Tyr Ala Lys Leu Gly Gly Ala Tyr Tyr Phe
        110             115             120

tca gag gcg aaa ggt ttt ggt gca gcc aaa tat aaa gat gat ggt gta          435
Ser Glu Ala Lys Gly Phe Gly Ala Ala Lys Tyr Lys Asp Asp Gly Val
    125             130             135

gtt ggt act gca ggt gca ggt ctt gag ttc ttc tta gat gat cat ctt          483
Val Gly Thr Ala Gly Ala Gly Leu Glu Phe Phe Leu Asp Asp His Leu
140             145             150             155

tct gct cgt cta gaa tat cag tac tac cat gat gta gaa cta aaa gac          531
Ser Ala Arg Leu Glu Tyr Gln Tyr Tyr His Asp Val Glu Leu Lys Asp
            160             165             170

aaa gat gtt cgc gca aat tgg gac act cac ttc tat ggt cta agc cta          579
Lys Asp Val Arg Ala Asn Trp Asp Thr His Phe Tyr Gly Leu Ser Leu
            175             180             185

gta tat agc tgg ggc gct cca gag cca gtt gca gaa cct gta tat gta          627
Val Tyr Ser Trp Gly Ala Pro Glu Pro Val Ala Glu Pro Val Tyr Val
        190             195             200

gat caa gtg tct gtt gcg act tta gaa gag ctt aaa ctg gcc gtt cca          675
Asp Gln Val Ser Val Ala Thr Leu Glu Glu Leu Lys Leu Ala Val Pro
        205             210             215

ttt gcc ttt gat agc tca tca att tct gca ggt gat gca gct aag cta          723
Phe Ala Phe Asp Ser Ser Ser Ile Ser Ala Gly Asp Ala Ala Lys Leu
220             225             230             235

gtt cca ttt gag cag cgt cta caa gag caa gat gca gca caa atc tat          771
Val Pro Phe Glu Gln Arg Leu Gln Glu Gln Asp Ala Ala Gln Ile Tyr
            240             245             250

gtt gtt ggt tat acc gat agc aaa ggt tct gaa gct tac aac cag aaa          819
Val Val Gly Tyr Thr Asp Ser Lys Gly Ser Glu Ala Tyr Asn Gln Lys
            255             260             265

ctg tct gag cgt cgt gca gat gct gtg gct gat gcg ctt cgt gca cat          867
Leu Ser Glu Arg Arg Ala Asp Ala Val Ala Asp Ala Leu Arg Ala His
        270             275             280

cta aat gtt gat ggt tct cgt att att gct gaa ggc cgt ggt gaa gca          915
Leu Asn Val Asp Gly Ser Arg Ile Ile Ala Glu Gly Arg Gly Glu Ala
        285             290             295

gat cca gtt gct tct aac caa aca gaa gaa ggt cgc gca cag aac cgt          963
Asp Pro Val Ala Ser Asn Gln Thr Glu Glu Gly Arg Ala Gln Asn Arg
300             305             310             315

cgt gtt gag atc gtt tct cct tca atc gat att gaa aca gta act caa          1011
Arg Val Glu Ile Val Ser Pro Ser Ile Asp Ile Glu Thr Val Thr Gln
            320             325             330

gtt ctt gct gaa cat cat cac cat cac cac tag ctcgagtcta gaggg          1059
Val Leu Ala Glu His His His His His His
        335             340
```

<210> 6

<211> 341
<212> PRT
<213> Photobacterium damselae subsp. piscicida

<400> 6

```
Met Ser Lys Val Arg Tyr Val Leu Pro Leu Ala Leu Leu Ile Ser Gly
1           5               10                  15

Val Ala Asn Ala Ala Ala Asp Asn Pro Trp Tyr Ala Gly Phe Arg Val
            20                25                  30

Gly Ala Thr His Tyr Asn Asp Ile Ser Val Asn Gly Val Asp Ser Asn
        35                40                  45

Ser Thr Phe Asp Arg Asp Asp Met Gly Gly Gly Leu Phe Ala Gly Tyr
    50                55                  60

Asn Val Thr Pro Trp Phe Ala Val Glu Thr Gly Tyr Thr Trp Leu Gly
65                70                  75                  80

Arg Ala Glu Phe Asp Asn Asn Tyr Lys Met Arg Val Asp Gln Gln Ala
            85                90                  95

Ile Asp Leu Val Gly Lys Phe Thr Trp His Ala Thr Asp Tyr Met Gly
            100               105                 110

Leu Tyr Ala Lys Leu Gly Gly Ala Tyr Tyr Phe Ser Glu Ala Lys Gly
        115               120                 125

Phe Gly Ala Ala Lys Tyr Lys Asp Asp Gly Val Val Gly Thr Ala Gly
    130               135                 140

Ala Gly Leu Glu Phe Phe Leu Asp Asp His Leu Ser Ala Arg Leu Glu
145               150                 155                 160

Tyr Gln Tyr Tyr His Asp Val Glu Leu Lys Asp Lys Asp Val Arg Ala
            165               170                 175

Asn Trp Asp Thr His Phe Tyr Gly Leu Ser Leu Val Tyr Ser Trp Gly
        180               185                 190

Ala Pro Glu Pro Val Ala Glu Pro Val Tyr Val Asp Gln Val Ser Val
    195               200                 205

Ala Thr Leu Glu Glu Leu Lys Leu Ala Val Pro Phe Ala Phe Asp Ser
    210               215                 220

Ser Ser Ile Ser Ala Gly Asp Ala Ala Lys Leu Val Pro Phe Glu Gln
```

225                230                235                240

Arg Leu Gln Glu Gln Asp Ala Ala Gln Ile Tyr Val Val Gly Tyr Thr
               245             250                255

Asp Ser Lys Gly Ser Glu Ala Tyr Asn Gln Lys Leu Ser Glu Arg Arg
             260             265             270

Ala Asp Ala Val Ala Asp Ala Leu Arg Ala His Leu Asn Val Asp Gly
         275             280             285

Ser Arg Ile Ile Ala Glu Gly Arg Gly Glu Ala Asp Pro Val Ala Ser
290                295            300

Asn Gln Thr Glu Glu Gly Arg Ala Gln Asn Arg Arg Val Glu Ile Val
305            310            315            320

Ser Pro Ser Ile Asp Ile Glu Thr Val Thr Gln Val Leu Ala Glu His
         325             330             335

His His His His His
         340

<210> 7
<211> 303
<212> DNA
<213> artificial sequence

<220>
<223> modified from P. damselae subsp. piscicida

<220>
<221> CDS
<222> (19)..(288)

<400> 7

```
agtgtggtgg aattcacc atg aat cgt aaa gtg act atc ctg gct ggt gtt        51
                     Met Asn Arg Lys Val Thr Ile Leu Ala Gly Val
                      1           5               10
```

```
att ctg agt gca act ctg atg ggt tgt tca agc tca agc gaa atg cag        99
Ile Leu Ser Ala Thr Leu Met Gly Cys Ser Ser Ser Ser Glu Met Gln
             15              20                  25
```

```
cag ctg act aac aaa gtt gac gca ctg tct gat cag gtg agc gct ctg       147
Gln Leu Thr Asn Lys Val Asp Ala Leu Ser Asp Gln Val Ser Ala Leu
             30              35                  40
```

```
cag gcc aac aga tca act agt ggc gct gtt aac gac gct cgt gca gca       195
Gln Ala Asn Arg Ser Thr Ser Gly Ala Val Asn Asp Ala Arg Ala Ala
             45              50                  55
```

```
tct gac gca gct tac cag gaa gca atg cgt gct aac cag cgc atc gac       243
Ser Asp Ala Ala Tyr Gln Glu Ala Met Arg Ala Asn Gln Arg Ile Asp
```

```
60                  65                  70                  75
```

```
aac atc cgt ggt tct tac act aag cat cat cac cat cac cac tag          288
Asn Ile Arg Gly Ser Tyr Thr Lys His His His His His His
                 80                  85
```

```
ctcgagtcta gaggg                                                     303
```

<210> 8
<211> 89
<212> PRT
<213> Photobacterium damselae subsp. piscicida

<400> 8

```
Met Asn Arg Lys Val Thr Ile Leu Ala Gly Val Ile Leu Ser Ala Thr
 1           5               10                  15
```

```
Leu Met Gly Cys Ser Ser Ser Ser Glu Met Gln Gln Leu Thr Asn Lys
             20                  25                  30
```

```
Val Asp Ala Leu Ser Asp Gln Val Ser Ala Leu Gln Ala Asn Arg Ser
             35                  40                  45
```

```
Thr Ser Gly Ala Val Asn Asp Ala Arg Ala Ala Ser Asp Ala Ala Tyr
     50                  55                  60
```

```
Gln Glu Ala Met Arg Ala Asn Gln Arg Ile Asp Asn Ile Arg Gly Ser
 65                  70                  75                  80
```

```
Tyr Thr Lys His His His His His His
                 85
```

<210> 9
<211> 1416

<212> DNA
<213> artificial sequence

<220>
<223> modified from P. damselae subsp. piscicida

<220>
<221> CDS
<222> (16)..(1401)

<400> 9

```
agtgtggtgg aattc atg aga aaa cct ctg ctg gtt ctg agt gca ctg gct      51
                  Met Arg Lys Pro Leu Leu Val Leu Ser Ala Leu Ala
                  1               5                   10

ctg ggt att agt tca gtt gtc acc ccc atg acc gca act gct gct ctg       99
Leu Gly Ile Ser Ser Val Val Thr Pro Met Thr Ala Thr Ala Ala Leu
        15                  20                  25

cct acc gcc gtt aat aat cag cag gtg cct agc ctg gct cca atg ctg      147
```

```
          Pro Thr Ala Val Asn Asn Gln Gln Val Pro Ser Leu Ala Pro Met Leu
              30                  35              40

          gag cag gtg aca cca gct gtg gtg agc att gca gtt gaa ggt aaa cac     195
          Glu Gln Val Thr Pro Ala Val Val Ser Ile Ala Val Glu Gly Lys His
              45                  50              55              60

          gtt tcc aaa cag cgc ctg cct gag gct tat cgc ttt ttc ttt ggt cca     243
          Val Ser Lys Gln Arg Leu Pro Glu Ala Tyr Arg Phe Phe Phe Gly Pro
                          65                  70              75

          gat ttt cct acc gag cag gtg cga gaa cag cca ttt cgg gga ctg ggc     291
          Asp Phe Pro Thr Glu Gln Val Arg Glu Gln Pro Phe Arg Gly Leu Gly
                      80                  85              90

          tct gga gtt atc att gac gct aaa aaa gga tat gtc gtt acc aat aac     339
          Ser Gly Val Ile Ile Asp Ala Lys Lys Gly Tyr Val Val Thr Asn Asn
                  95                  100             105

          cac gtt att gat ggc gca gat acc atc aaa gtg cag ctg tct gac gga     387
          His Val Ile Asp Gly Ala Asp Thr Ile Lys Val Gln Leu Ser Asp Gly
              110                 115             120

          cgt gag atc aaa gca aag ctg ctg ggt acc gat aaa atg tct gat att     435
          Arg Glu Ile Lys Ala Lys Leu Leu Gly Thr Asp Lys Met Ser Asp Ile
              125             130             135             140

          gct ctg ctg cag ctg gaa gat gcc aaa gat ctg acc gct atc aaa ctc     483
          Ala Leu Leu Gln Leu Glu Asp Ala Lys Asp Leu Thr Ala Ile Lys Leu
                          145                 150             155

          gct gat tct gat aat ctg cgc gtg ggc gat ttc gcc gtt gct atc ggt     531
          Ala Asp Ser Asp Asn Leu Arg Val Gly Asp Phe Ala Val Ala Ile Gly
                          160                 165             170

          aac cca ttt ggc ctg gga cag aca gtg aca tca ggt att gtt tcc gct     579
          Asn Pro Phe Gly Leu Gly Gln Thr Val Thr Ser Gly Ile Val Ser Ala
                      175                 180             185

          ctg ggt cgt agt ggt ctg aat atc gag aac ttc gaa aac ttt atc cag     627
          Leu Gly Arg Ser Gly Leu Asn Ile Glu Asn Phe Glu Asn Phe Ile Gln
              190                 195             200

          act gat gct cca atc aat agc ggt aac tca ggt ggt gcc ctg gtg aac     675
          Thr Asp Ala Pro Ile Asn Ser Gly Asn Ser Gly Gly Ala Leu Val Asn
          205                 210             215             220

          ctg aac ggt gag ctg att ggt att aat acc gct att ctg gct cct aac     723
          Leu Asn Gly Glu Leu Ile Gly Ile Asn Thr Ala Ile Leu Ala Pro Asn
                          225                 230             235

          ggt ggt aac gtc ggt atc ggt ttt gca atc cct gct aat atg gtg aaa     771
          Gly Gly Asn Val Gly Ile Gly Phe Ala Ile Pro Ala Asn Met Val Lys
                      240                 245             250

          aac ctg aca gat cag ctg atc gaa ttt ggt cag gtt aag cgc ggt gtt     819
          Asn Leu Thr Asp Gln Leu Ile Glu Phe Gly Gln Val Lys Arg Gly Val
                      255                 260             265

          ctg ggt gtt act ggt ggt gag ctg acc tct gag ctg gct gaa acc ttt     867
          Leu Gly Val Thr Gly Gly Glu Leu Thr Ser Glu Leu Ala Glu Thr Phe
              270                 275             280
```

25

```
ggt tat aaa aca aat cac ggc gct ttt gtg aac cag gtg ctg cct gaa        915
Gly Tyr Lys Thr Asn His Gly Ala Phe Val Asn Gln Val Leu Pro Glu
285             290             295             300

ggc tct gct gca aaa gca ggt ctg aaa gca ggt gat att att gtt tca        963
Gly Ser Ala Ala Lys Ala Gly Leu Lys Ala Gly Asp Ile Ile Val Ser
        305             310             315

gtc aat aat aag ccc att cgt act ttc agt gaa ctg cgc gca aaa att       1011
Val Asn Asn Lys Pro Ile Arg Thr Phe Ser Glu Leu Arg Ala Lys Ile
        320             325             330

gtg act ctg ggt gct ggt aag aaa gtg aca ctg ggc ctg att cgc gat       1059
Val Thr Leu Gly Ala Gly Lys Lys Val Thr Leu Gly Leu Ile Arg Asp
        335             340             345

ggt aaa gag ctg aat gtt cct gtg act ctg gaa gca gca aaa cag act       1107
Gly Lys Glu Leu Asn Val Pro Val Thr Leu Glu Ala Ala Lys Gln Thr
350             355             360

cag gtt aaa gct gat gat ctg cac gaa agc ctg gcc ggt gct gaa ttt       1155
Gln Val Lys Ala Asp Asp Leu His Glu Ser Leu Ala Gly Ala Glu Phe
365             370             375             380

gct aat act agc cca gaa gat aaa gtt cac gga gtt aaa gtg acc gag       1203
Ala Asn Thr Ser Pro Glu Asp Lys Val His Gly Val Lys Val Thr Glu
            385             390             395

ctg aac aag cag tct att gct gct cgc tat ggc ctg aag aag ggc gat       1251
Leu Asn Lys Gln Ser Ile Ala Ala Arg Tyr Gly Leu Lys Lys Gly Asp
        400             405             410

atc att att ggt ctg aac cgt cag cca att aaa aac ctg ggc gaa ctg       1299
Ile Ile Ile Gly Leu Asn Arg Gln Pro Ile Lys Asn Leu Gly Glu Leu
        415             420             425

cgt aag gct ctg gag aaa aaa cca aac gtg ctg gca atg gaa gtt aaa       1347
Arg Lys Ala Leu Glu Lys Lys Pro Asn Val Leu Ala Met Glu Val Lys
430             435             440

cgt ggt gat agc gtg ctg tat ctg atc atg cgt cat cat cac cat cac       1395
Arg Gly Asp Ser Val Leu Tyr Leu Ile Met Arg His His His His His
445             450             455             460

cac tag ctcgagtcta gaggg                                              1416
His
```

<210> 10
<211> 461
<212> PRT
<213> Photobacterium damselae subsp. piscicida

<400> 10

```
Met Arg Lys Pro Leu Leu Val Leu Ser Ala Leu Ala Leu Gly Ile Ser
1               5               10              15


Ser Val Val Thr Pro Met Thr Ala Thr Ala Ala Leu Pro Thr Ala Val
            20              25              30
```

26

```
Asn Asn Gln Gln Val Pro Ser Leu Ala Pro Met Leu Glu Gln Val Thr
        35              40              45

Pro Ala Val Val Ser Ile Ala Val Glu Gly Lys His Val Ser Lys Gln
        50              55              60

Arg Leu Pro Glu Ala Tyr Arg Phe Phe Phe Gly Pro Asp Phe Pro Thr
65              70              75              80

Glu Gln Val Arg Glu Gln Pro Phe Arg Gly Leu Gly Ser Gly Val Ile
            85              90              95

Ile Asp Ala Lys Lys Gly Tyr Val Val Thr Asn Asn His Val Ile Asp
            100             105             110

Gly Ala Asp Thr Ile Lys Val Gln Leu Ser Asp Gly Arg Glu Ile Lys
            115             120             125

Ala Lys Leu Leu Gly Thr Asp Lys Met Ser Asp Ile Ala Leu Leu Gln
        130             135             140

Leu Glu Asp Ala Lys Asp Leu Thr Ala Ile Lys Leu Ala Asp Ser Asp
145             150             155             160

Asn Leu Arg Val Gly Asp Phe Ala Val Ala Ile Gly Asn Pro Phe Gly
                165             170             175

Leu Gly Gln Thr Val Thr Ser Gly Ile Val Ser Ala Leu Gly Arg Ser
            180             185             190

Gly Leu Asn Ile Glu Asn Phe Glu Asn Phe Ile Gln Thr Asp Ala Pro
        195             200             205

Ile Asn Ser Gly Asn Ser Gly Gly Ala Leu Val Asn Leu Asn Gly Glu
        210             215             220

Leu Ile Gly Ile Asn Thr Ala Ile Leu Ala Pro Asn Gly Gly Asn Val
225             230             235             240

Gly Ile Gly Phe Ala Ile Pro Ala Asn Met Val Lys Asn Leu Thr Asp
            245             250             255

Gln Leu Ile Glu Phe Gly Gln Val Lys Arg Gly Val Leu Gly Val Thr
        260             265             270

Gly Gly Glu Leu Thr Ser Glu Leu Ala Glu Thr Phe Gly Tyr Lys Thr
        275             280             285
```

```
Asn His Gly Ala Phe Val Asn Gln Val Leu Pro Glu Gly Ser Ala Ala
    290                 295                 300

Lys Ala Gly Leu Lys Ala Gly Asp Ile Ile Val Ser Val Asn Asn Lys
305                 310                 315                 320

Pro Ile Arg Thr Phe Ser Glu Leu Arg Ala Lys Ile Val Thr Leu Gly
                325                 330                 335

Ala Gly Lys Lys Val Thr Leu Gly Leu Ile Arg Asp Gly Lys Glu Leu
            340                 345                 350

Asn Val Pro Val Thr Leu Glu Ala Ala Lys Gln Thr Gln Val Lys Ala
            355                 360                 365

Asp Asp Leu His Glu Ser Leu Ala Gly Ala Glu Phe Ala Asn Thr Ser
    370                 375                 380

Pro Glu Asp Lys Val His Gly Val Lys Val Thr Glu Leu Asn Lys Gln
385                 390                 395                 400

Ser Ile Ala Ala Arg Tyr Gly Leu Lys Lys Gly Asp Ile Ile Ile Gly
                405                 410                 415

Leu Asn Arg Gln Pro Ile Lys Asn Leu Gly Glu Leu Arg Lys Ala Leu
            420                 425                 430

Glu Lys Lys Pro Asn Val Leu Ala Met Glu Val Lys Arg Gly Asp Ser
            435                 440                 445

Val Leu Tyr Leu Ile Met Arg His His His His His
    450                 455                 460
```

<210> 11
<211> 1056
<212> DNA
<213> artificial sequence

<220>
<223> modified from P. damselae subsp. piscicida

<220>
<221> CDS
<222> (16)..(1041)

<400> 11

```
agtgtggtgg aattc atg tct aaa gtt cgt tat gtt ctg ccc ctg gca ctg          51
               Met Ser Lys Val Arg Tyr Val Leu Pro Leu Ala Leu
               1               5               10
```

```
ctg att tca ggt gtc gct aac gca gca gca gat aac cca tgg tat gct          99
Leu Ile Ser Gly Val Ala Asn Ala Ala Ala Asp Asn Pro Trp Tyr Ala
        15              20              25

ggt ttc cga gtt ggt gct act cac tat aat gat att tct gtg aat ggt         147
Gly Phe Arg Val Gly Ala Thr His Tyr Asn Asp Ile Ser Val Asn Gly
    30              35              40

gtg gac agt aat tct aca ttt gac cgc gat gat atg ggc ggt ggt ctg         195
Val Asp Ser Asn Ser Thr Phe Asp Arg Asp Asp Met Gly Gly Gly Leu
45              50              55              60

ttt gca ggt tac aac gtg act cct tgg ttc gct gtt gaa act ggc tac         243
Phe Ala Gly Tyr Asn Val Thr Pro Trp Phe Ala Val Glu Thr Gly Tyr
                65              70              75

act tgg ctg ggc cgt gct gag ttt gat aat aac tac aaa atg cga gtt         291
Thr Trp Leu Gly Arg Ala Glu Phe Asp Asn Asn Tyr Lys Met Arg Val
            80              85              90

gat cag cag gct atc gat ctg gtt ggt aaa ttt aca tgg cac gca aca        339
Asp Gln Gln Ala Ile Asp Leu Val Gly Lys Phe Thr Trp His Ala Thr
        95              100             105

gac tat atg ggt ctg tat gct aaa ctg ggt ggt gca tac tac ttc tca        387
Asp Tyr Met Gly Leu Tyr Ala Lys Leu Gly Gly Ala Tyr Tyr Phe Ser
        110             115             120

gag gct aaa ggt ttt ggt gca gcc aaa tat aaa gat gat ggt gtg gtt        435
Glu Ala Lys Gly Phe Gly Ala Ala Lys Tyr Lys Asp Asp Gly Val Val
125             130             135             140

ggt act gca ggt gca ggt ctg gag ttc ttc ctg gat gat cac ctg tct        483
Gly Thr Ala Gly Ala Gly Leu Glu Phe Phe Leu Asp Asp His Leu Ser
                145             150             155

gct cgt ctg gaa tat cag tac tac cac gat gtg gaa ctg aaa gac aaa        531
Ala Arg Leu Glu Tyr Gln Tyr Tyr His Asp Val Glu Leu Lys Asp Lys
            160             165             170

gat gtt cgc gca aat tgg gac act cac ttc tat ggt ctg agc ctg gtg        579
Asp Val Arg Ala Asn Trp Asp Thr His Phe Tyr Gly Leu Ser Leu Val
            175             180             185

tat agc tgg ggc gct cca gag cca gtt gca gaa cct gtg tat gtg gat        627
Tyr Ser Trp Gly Ala Pro Glu Pro Val Ala Glu Pro Val Tyr Val Asp
        190             195             200

cag gtg tct gtt gct act ctg gaa gag ctg aaa ctg gcc gtt cca ttt        675
Gln Val Ser Val Ala Thr Leu Glu Glu Leu Lys Leu Ala Val Pro Phe
205             210             215             220

gcc ttt gat agc tca tca att tct gca ggt gat gca gct aag ctg gtt        723
Ala Phe Asp Ser Ser Ser Ile Ser Ala Gly Asp Ala Ala Lys Leu Val
                225             230             235

cca ttt gag cag cgt ctg cag gag cag gat gca gca cag atc tat gtt        771
Pro Phe Glu Gln Arg Leu Gln Glu Gln Asp Ala Ala Gln Ile Tyr Val
            240             245             250

gtt ggt tat acc gat agc aaa ggt tct gaa gct tac aac cag aaa ctg        819
Val Gly Tyr Thr Asp Ser Lys Gly Ser Glu Ala Tyr Asn Gln Lys Leu
```

```
                 255                    260                    265

     tct gag cgt cgt gca gat gct gtg gct gat gct ctg cgt gca cac ctg      867
     Ser Glu Arg Arg Ala Asp Ala Val Ala Asp Ala Leu Arg Ala His Leu
         270             275             280

     aat gtt gat ggt tct cgt att att gct gaa ggc cgt ggt gaa gca gat      915
     Asn Val Asp Gly Ser Arg Ile Ile Ala Glu Gly Arg Gly Glu Ala Asp
     285             290             295             300

     cca gtt gct tct aac cag aca gaa gaa ggt cgc gca cag aac cgt cgt      963
     Pro Val Ala Ser Asn Gln Thr Glu Glu Gly Arg Ala Gln Asn Arg Arg
             305             310             315

     gtt gag atc gtt tct cct tca atc gat att gaa aca gtg act cag gtt     1011
     Val Glu Ile Val Ser Pro Ser Ile Asp Ile Glu Thr Val Thr Gln Val
             320             325             330

     ctg gct gaa cat cat cac cat cac cac tag ctcgagtcta gaggg           1056
     Leu Ala Glu His His His His His His
             335             340
```

<210> 12
<211> 341
<212> PRT
<213> Photobacterium damselae subsp. piscicida

<400> 12

```
Met Ser Lys Val Arg Tyr Val Leu Pro Leu Ala Leu Leu Ile Ser Gly
1               5               10                  15

Val Ala Asn Ala Ala Ala Asp Asn Pro Trp Tyr Ala Gly Phe Arg Val
        20              25                      30

Gly Ala Thr His Tyr Asn Asp Ile Ser Val Asn Gly Val Asp Ser Asn
        35              40                  45

Ser Thr Phe Asp Arg Asp Asp Met Gly Gly Gly Leu Phe Ala Gly Tyr
    50              55                  60

Asn Val Thr Pro Trp Phe Ala Val Glu Thr Gly Tyr Thr Trp Leu Gly
65              70                  75                      80

Arg Ala Glu Phe Asp Asn Asn Tyr Lys Met Arg Val Asp Gln Gln Ala
            85                  90                  95

Ile Asp Leu Val Gly Lys Phe Thr Trp His Ala Thr Asp Tyr Met Gly
        100                 105                 110

Leu Tyr Ala Lys Leu Gly Gly Ala Tyr Tyr Phe Ser Glu Ala Lys Gly
        115                 120                 125

Phe Gly Ala Ala Lys Tyr Lys Asp Asp Gly Val Val Gly Thr Ala Gly
```

```
            130                    135                          140


        Ala Gly Leu Glu Phe Phe Leu Asp Asp His Leu Ser Ala Arg Leu Glu
        145             150             155                 160


        Tyr Gln Tyr Tyr His Asp Val Glu Leu Lys Asp Lys Asp Val Arg Ala
                        165             170                 175


        Asn Trp Asp Thr His Phe Tyr Gly Leu Ser Leu Val Tyr Ser Trp Gly
                    180             185                 190


        Ala Pro Glu Pro Val Ala Glu Pro Val Tyr Val Asp Gln Val Ser Val
                    195             200                 205


        Ala Thr Leu Glu Glu Leu Lys Leu Ala Val Pro Phe Ala Phe Asp Ser
            210             215                 220


        Ser Ser Ile Ser Ala Gly Asp Ala Ala Lys Leu Val Pro Phe Glu Gln
        225             230             235                 240


        Arg Leu Gln Glu Gln Asp Ala Ala Gln Ile Tyr Val Val Gly Tyr Thr
                        245             250                 255


        Asp Ser Lys Gly Ser Glu Ala Tyr Asn Gln Lys Leu Ser Glu Arg Arg
                    260             265                 270


        Ala Asp Ala Val Ala Asp Ala Leu Arg Ala His Leu Asn Val Asp Gly
                    275             280                 285


        Ser Arg Ile Ile Ala Glu Gly Arg Gly Glu Ala Asp Pro Val Ala Ser
            290             295                 300


        Asn Gln Thr Glu Glu Gly Arg Ala Gln Asn Arg Arg Val Glu Ile Val
        305             310             315                 320


        Ser Pro Ser Ile Asp Ile Glu Thr Val Thr Gln Val Leu Ala Glu His
                        325             330                 335


        His His His His His
                        340
```

<210> 13
<211> 30
<212> DNA
<213> artificial sequence

<220>
<223> ppa1-forward primer

<400> 13
cggaattcac catgaatcgt aaagtaacta     30

<210> 14
<211> 26
<212> DNA
<213> artificial sequence

<220>
<223> ppa1-reverse primer

<400> 14
ccgctcgagc ttagtgtaag aaccac     26

<210> 15
<211> 30
<212> DNA
<213> artificial sequence

<220>
<223> ppa2-forward primer

<400> 15
cggaattcac catgagaaaa cctctgcttg     30

<210> 16
<211> 26
<212> DNA
<213> artificial sequence

<220>
<223> ppa2-reverse primer

<400> 16
ccgctcgaga cgcatgatta aataca     26

<210> 17
<211> 30
<212> DNA
<213> artificial sequence

<220>
<223> ppars1-forward primer

<400> 17
cggaattcac catgtctaaa gttcgttatg     30

<210> 18
<211> 26
<212> DNA
<213> artificial sequence

<220>
<223> ppars1-reverse primer

<400> 18
ccgctcgagt tcagcaagaa cttgag     26